Europäisches Patentamt

**European Patent Office**

Office européen des brevets

(11) Numéro de publication: **0 121 447**
A1

## (12) DEMANDE DE BREVET EUROPEEN

(21) Numéro de dépôt: **84400294.9**

(22) Date de dépôt: **14.02.84**

(51) Int. Cl.³: **A 61 F 1/00**

(30) Priorité: **01.03.83 FR 8303315**

(43) Date de publication de la demande:
**10.10.84 Bulletin 84/41**

(84) Etats contractants désignés:
**AT BE CH DE GB IT LI LU NL SE**

(71) Demandeur: **Franceschi, Claude**
**21, quai Alphonse Le Gallo**
**F-92100 Boulogne(FR)**

(72) Inventeur: **Franceschi, Claude**
**21, quai Alphonse Le Gallo**
**F-92100 Boulogne(FR)**

(74) Mandataire: **Peuscet, Jacques**
**3, Square de Maubeuge**
**F-75009 Paris(FR)**

(54) **Filtre contre les embolies.**

(57) Filtre contre les embolies, constitué d'un fil prédéformé avec des ondulations principales et secondaires dans des plan différents. Pour la mise en place le filtre reprend temporairement une position rectiligne dans un cathéter.

FIG. 1

EP 0 121 447 A1

1

# FILTRE CONTRE LES EMBOLIES

La présente invention a trait à un filtre contre les embolies ainsi qu'un procédé pour la mise en place de ce filtre dans un vaisseau. On sait que les embolies pulmonaires, particulièrement dangereuses, sont provoquées la plupart du temps par des emboles provenant de thromboses ou de phlébites du membre inférieur d'où ils se dirigent vers l'artère pulmonaire par la veine cave inférieure et la circulation cardiaque droite.

Du fait que les risques de thrombo-phlébites sont souvent liés à des circonstances médicales ou hospitalières (alitement prolongé, accouchement) et que de plus les thrombo-phlébites notamment des membres inférieurs, sont en général cliniquement décelables puis localisables par angiographie, on a déjà proposé de prévenir la remontée d'un embole dans la veine cave inférieure par la mise en place d'un obstacle.

L'un de ces obstacles est constitué par le clips ADAMS DE WEESE qui présente une forme de clips muni de plusieurs larges échancrures que l'on met en place autour de la veine cave inférieure qui se trouve ainsi pincée et déformée de façon à former plusieurs passages étroits. Cette technique a le grave inconvénient de nécessiter une laparotomie. De plus elle tend à augmenter la résistance à l'écoulement et par conséquent la stase veineuse.

Un autre dispositif connu est le filtre de MOBIN-UDDIN qui est constitué par un filtre sous forme d'un disque muni d'une pluralité de perforations avec, à sa périphérie, des ergots destinés à se planter radialement dans la paroi veineuse. Ce filtre est initialement replié à la façon d'un parapluie afin de pouvoir être mis en place par une veine jugulaire puis ouvert in situ grâce à un guide tubulaire que l'on retire.

Un tel dispositif présente l'inconvénient d'être difficile à mettre en place en raison de son encombrement, même à l'état replié, de risquer de se boucher, entraînant par là un syndrome de la veine cave inférieure, et même de risquer de migrer et de provoquer d'importantes lésions.

Une troisième technique consiste à utiliser un filtre de GREENFIELD, constitué d'une pluralité de fils de forme ondulée se répartissant suivant un cône à partir d'un sommet commun. Un tel filtre est également difficile à introduire en raison de sa taille et risque de se mettre de travers et de migrer.

En outre, dans le premier cas, on crée à l'emplacement du clips une zone rigide au niveau de la veine cave. Dans les deux autres cas, on crée une contrainte constante sur les parois.

La présente invention se propose de remédier à ces inconvénients et de fournir un filtre contre les embolies susceptible d'être introduit facilement par la voie de diagnostic, c'est-à-dire la voie permettant la localisation du caillot par cathétérisme sous radioscopie, et cela sans nécessiter un guide de diamètre important.

Un autre objectif de l'invention est de pouvoir mettre en place le filtre en évitant tout risque de mauvais positionnement du filtre dans le vaisseau.

Un autre objectif de l'invention est de réduire l'agression de la paroi vasculaire par le filtre.

Un autre objectif encore de l'invention est de permettre d'assurer une filtration du débit sanguin sans pratiquement d'augmentation de la perte de charge et sans risque de voir le filtre se boucher.

Un autre objectif de l'invention est de réduire voire de supprimer tout risque de déplacement secondaire.

Un autre objectif encore de l'invention est d'éviter que le filtre puisse être déplacé par un caillot ou embole exerçant un effort important.

L'un des avantages importants du filtre selon l'invention est ainsi de pouvoir être mis en place dans des vaisseaux de taille plus réduite, par exemple la veine

3

iliaque en amont de laquelle on a détecté un caillot.

L'invention a pour objet un filtre contre les embolies caractérisé par le fait qu'il se présente sous la forme d'un fil élastique pré-déformé, susceptible d'être amené dans une position rectiligne puis lorsqu'il est libéré, de reprendre une forme correspondant à sa pré-déformation, la pré-déformation du fil, lui donnant une forme ondulée avec des ondulations successives orientées dans des plans différents de l'espace, les deux extrémités du fil étant, de préférence, munies de petites pointes destinées à se prendre dans la paroi vasculaire.

L'amplitude des pré-déformations est telle que selon une caractéristique de l'invention, le fil vient par certains au moins des sommets d'ondulations s'appliquer élastiquement contre la paroi vasculaire qui tend alors à déformer.

Dans une forme de réalisation particulièrement préférée, le fil comporte au moins deux ondulations successives de préférence dans un même plan, d'amplitude supérieure au diamètre du vaisseau qui doit le recevoir, lesdites ondulations formant ainsi quatre branches et trois sommets; chaque branche présente une pluralité d'ondulations secondaires de moindre amplitude dans un plan sensiblement perpendiculaire au plan de l'ondulation principale, lesdites ondulations secondaires étant, de préférence, disposées de façon telle que, lorsqu'elles sont vues dans le sens axial, c'est-à-dire dans le sens de la circulation du sang, les sommets des ondulations secondaires sur les branches principales se trouvent alternativement dans un même plan que les zones inter-sommet des branches d'ondulations secondaires sur d'autres branches principales.

Ainsi, le filtre présente, lorsqu'il est vu dans le sens axial, l'apparence d'un réseau relativement aplati dont on comprend qu'il forme un obstacle parfait pour le passage d'un embole sans constituer la moindre gêne d'écoulement.

Dans une telle forme de réalisation, le filtre lorsqu'il est mis en place, par exemple dans une veine cave, -----tend à aplatir quelque peu le vaisseau pour lui donner une section sensiblement ovale dont le grand diamètre répond à l'amplitude des grandes ondulations et dont le petit diamètre répond à l'amplitude des ondulations secondaires, en tenant compte chaque fois de l'élasticité propre du fil constituant le filtre et de celle de la paroi vasculaire.

Le fil utilisé présente, de préférence, un diamètre compris entre 1 mm et 0,3 mm. Il est réalisé, par exemple, en un alliage métallique nickel-titane, tel que par exemple le fil vendu sous la dénomination Nitinol Activ-Arch par la Société de droit américain "Unitek Corporation". Il se caractérise par le fait qu'il peut être remis à l'état rectiligne tout en reprenant, dès qu'il est libéré, l'ensemble des déformations permanentes qu'il a préalablement subies.

Le cas échéant, le fil peut être recouvert d'une couche de polycarbonate si l'on veut éliminer totalement le risque de production de filaments de fibrine.

Pour mettre en place le filtre selon l'invention, on choisit -------------------------------------------- un filtre dont l'amplitude des ondulations est supérieure à la dimension du vaisseau qui reçoit le filtre ; on introduit dans un vaisseau, par exemple la veine jugulaire, un fil directeur ; on enfile ensuite sur le fil directeur un cathéter ; on retire le fil directeur ; on enfile dans l'extrémité du cathéter le filtre sous forme allongée puis un câble-poussoir qui repousse le fil le long du cathéter et , une fois le filtre mis en place, on ramène en arrière le cathéter en maintenant le câble-poussoir (jusqu'à libération du fil qui tend à reprendre sa forme ondulée) après quoi on retire le cathéter et le câble poussoir; toutes ces opérations s'effectuent, bien entendu, sous radioscopie.

5

D'autres avantages et caractéristiques de l'invention apparaîtront à la lecture de la description suivante faite à titre d'exemple non limitatif et se référant au dessin annexé dans lequel :

- la figure 1 représente une vue en élévation du filtre selon l'invention placé dans une veine ;

- la figure 2 représente une vue de profil de ce filtre.

Le filtre représenté sur les figures est réalisé en un alliage nickel-titane dont la caractéristique, connue, est de conserver la mémoire des déformations permanentes subies même lorsqu'il est ramené à l'état rectiligne. La longueur du fil est par exemple de 15 cm et son diamètre de 0,5 mm.

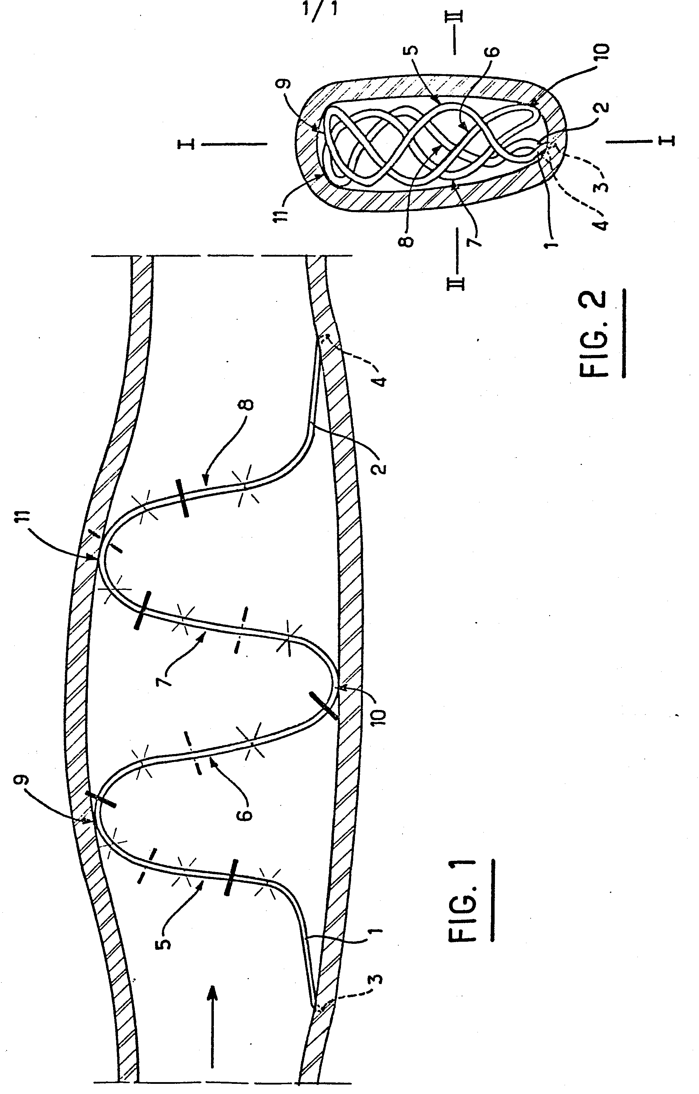

Le fil a été déformé de façon à adopter spontanément la forme représentée sur les figures 1 et 2. A vrai dire, du fait que le fil est représenté dans une veine qu'il a déformée, et dont la résistance à la déformation agit sur le fil celui-ci n'a pas retrouvé toute l'amplitude de ses ondulations ; dans un but de simplicité, on considèrera que la forme à l'état de repos du fil est celle représentée sur les figures.

Comme on le voit, le fil se termine par deux extrémités 1, 2 recourbées en forme d'ergots pointus 3, 4 qui sont destinés, sous l'effet de l'élasticité du fil, à pénétrer dans la paroi vasculaire. Entre les extrémités 1 et 2 le fil présente deux ondulations principales formant les quatre branches 5, 6, 7, 8 réunies par trois sommets 9, 10 et 11. Il va de soi que le fil pourrait éventuellement ne comporter que deux ou trois branches ou, au contraire, plus de quatre branches avec un nombre d'ondulations correspondant. Les différentes ondulations principales peuvent être égales entre elles mais peuvent également varier suivant la longueur axiale totale que l'on veut donner au fil à l'état de repos dans la direction de la flèche représentant

6

le sens du courant.

Lorsqu'on le considère non plus en élévation mais en profil, on voit que chacune des branches possède des ondulations secondaires et l'on a représenté sur la figure 1, par des croix, l'emplacement où le fil passe dans son plan médian I-I, par des traits transversaux les sommets des ondulations secondaires situés à droite du plan I-I sur la figure 2 et par des traits transversaux interrompus, les sommets des ondulations secondaires situés à gauche de ce plan.

Le nombre et l'amplitude de ces ondulations peuvent évidemment varier mais il est préféré que l'amplitude des ondulations secondaires ne soit pas supérieure à 30 % de l'amplitude des ondulations principales.

On voit d'ailleurs, dans la forme de réalisation préférée de l'invention qui a été représentée, que le plan II-II, parallèle à la flèche et perpendiculaire au plan I-I, contient un sommet représenté par un trait plein, situé sur la branche 5 et situé à droite du plan I-I sur la figure 2, un passage dans le plan I-I de la branche 6, un sommet situé de l'autre côté de ce plan de la branche 7 et un nouveau point de passage dans le plan II-II de la branche 8. Une alternance similaire est également retrouvée pour les autres plans parallèles au plan II-II passant par les sommets respectifs des ondulations secondaires.

Grâce à cette forme on obtient, en vue de profil, une apparence de réseau, qui présente la particularité de ne former qu'une gêne extrêmement faible à l'écoulement et de n'entraîner, par conséquent, qu'une perte de charge minime tout en constituant un obstacle infranchissable au passage d'un embole de taille suffisante pour être dangereux. Un tel embole se trouverait alors pris et immobilisé par le filtre tout en restant balayé par le flux sanguin, ce qui permet une action particulièrement efficace des médications anti-thrombotiques ou thrombolytiques.

7

Comme on l'a vu, l'introduction peut être effectuée extrêment facilement par la voie vasculaire de recherche du caillot, le plus souvent par l'intermédiaire de la
veine jugulaire ou fémorale.--------------------------------
-------------------------------------------------- Lors de la mise en
place,le fil formant le filtre, étant introduit et emprisonné à l'intérieur de la lumière d'un cathéter, prend une
forme allongée et est repoussé à l'intérieur du cathéter
pour en être extrait à l'emplacement choisi. Cette mise en
place permet d'utiliser un cathéter de très petit diamètre,
juste suffisant pour contenir et guider le fil de sorte que
la mise en place est particulièrement facile, permettant
notamment d'atteindre des vaisseaux d'assez petit diamètre
et d'éviter la mise en place du filtre dans la veine cave.

Du fait que le cathéter présente obligatoirement
une forme sensiblement axiale dans les vaisseaux dans
lesquels il est disposé, le fil lorsqu'il est libéré du cathéter se trouve dans une position axiale et va donc être
convenablement positionné d'emblée. En reprenant sa forme
initiale déformée,le fil formant le filtre va, par ses extrémités et sommets d'ondulations prendre contact avec la paroi
vasculaire qu'il va déformer de sorte que le vaisseau se
trouve ovalisé, comme on le voit sur le dessin. Les ergots
d'accrochage 3, 4 pénètrent légèrement dans la paroi vasculaire mais, en raison de la pression élastique exercée par
les ondulations du filtre sur la paroi vasculaire, le maintien reste convenablement assuré, même en cas de décrochage
des ergots d'accrochage. De toute façon l'agression sur la
paroi vasculaire se trouve considérablement limitée par
rapport aux techniques antérieures.

Les déplacements vers le haut, vers le bas ou les
déplacements transversaux se trouvent également empêchés par
la position prise par le filtre. Celui-ci, malgré son
maintien n'augmente pas la résistance de passage du flux
sanguin tout en arrêtant efficacement les emboles comme cela

8

a été vu ci-dessus. En raison de l'accrochage et de la tension élastique des sommets contre une seule paroi vasculaire, un embol de grande dimension formé sur le filtre est incapable de le déplacer. Enfin, en raison des grandes possibilités de déformation élastique que possède le filtre, on peut comprendre qu'il peut s'adapter à d'assez grandes augmentations de diamètre du vaisseau dans lequel il est placé sans pour cela se déplacer.

Bien entendu le filtre selon l'invention peut faire l'objet de nombreuses variantes et la forme relativement aplatie, munie d'ondulations principale sur lesquelles sont disposées des ondulations secondaires, peut être variée dans de larges proportions.

9

## REVENDICATIONS

1. Filtre contre les embolies, caractérisé par le fait qu'il est constitué d'un fil élastique pré-déformé, susceptible d'être amené dans une position rectiligne puis, lorsqu'il est libéré, de reprendre une forme correspondant à sa pré-déformation, ladite pré-déformation lui donnant une forme ondulée, avec des ondulations successives orientées dans des plans différents de l'espace.

2. Filtre selon la revendication 1, caractérisé par le fait que les deux extrémités du fil sont recourbées en forme de petits ergots (3, 4).

3. Filtre selon l'une des revendications 1 et 2, caractérisé par le fait qu'il comporte au moins deux ondulations principales successives formant des branches (5, 6, 7, 8) et des sommets (9, 10, 11), chaque branche présentant une pluralité d'ondulations secondaires dans un plan sensiblement perpendiculaire au plan de la branche principale.

4. Filtre selon la revendication 3, caractérisé par le fait qu'il comporte deux ondulations principales formant quatre branches et trois sommets.

5. Filtre selon l'une des revendications 3 et 4, caractérisé par le fait que, dans le sens axial du filtre, les sommets des ondulations secondaires sur les branches principales se trouvent alternativement dans un même plan (II-II) que les zones inter-sommets des branches d'ondulations secondaires appartenant à d'autres branches principales.

6. Filtre selon l'une quelconque des revendications 1 à 5, caractérisé par le fait que le fil est réalisé en un alliage nickel-titane.

7. Filtre selon l'une quelconque des revendications 1 à 6, caractérisé par le fait que le fil est recouvert d'une couche de polycarbonate.

FIG. 1

FIG. 2

1/1

0121447

Office européen
des brevets

**0121447**
Numéro de la demande

RAPPORT DE RECHERCHE EUROPEENNE

EP 84 40 0294

## DOCUMENTS CONSIDERES COMME PERTINENTS

| Catégorie | Citation du document avec indication, en cas de besoin, des parties pertinentes | Revendication concernée | CLASSEMENT DE LA DEMANDE (Int. Cl. 3) |
|---|---|---|---|
| A | DE-A-3 203 410 (HEINKE) <br> * Résumé; figures * | 1,3 | A 61 F 1/00 |
| A | MEDICAL AND BIOLOGICAL ENGINEERING, novembre 1976, pages 629-635, New York, USA; J. DRILLER et al.: "New percutaneous caval filter device for pulmonary thromboembolism" | | |
| A | US-A-3 334 629 (COHN) | | |
| A | US-A-3 540 431 (KAZI BOMIN-UDDIN) | | |

---

| DOMAINES TECHNIQUES RECHERCHES (Int. Cl. 3) |
|---|
| A 61 F |

Le présent rapport de recherche a été établi pour toutes les revendications

| Lieu de la recherche | Date d'achèvement de la recherche | Examinateur |
|---|---|---|
| LA HAYE | 13-06-1984 | STEENBAKKER J. |

CATEGORIE DES DOCUMENTS CITES

X : particulièrement pertinent à lui seul
Y : particulièrement pertinent en combinaison avec un autre document de la même catégorie
A : arrière-plan technologique
O : divulgation non-écrite
P : document intercalaire

T : théorie ou principe à la base de l'invention
E : document de brevet antérieur, mais publié à la date de dépôt ou après cette date
D : cité dans la demande
L : cité pour d'autres raisons

& : membre de la même famille, document correspondant

OEB Form 1503. 03.82